(19) Europäisches Patentamt
European Patent Office
Office européen des brevets

(11) Veröffentlichungsnummer: **0 089 598**
**B1**

(12) **EUROPÄISCHE PATENTSCHRIFT**

(45) Veröffentlichungstag der Patentschrift: **08.01.86**

(51) Int. Cl.⁴: **G 01 N 33/546**

(21) Anmeldenummer: **83102523.4**

(22) Anmeldetag: **15.03.83**

(54) **Anti-Streptolysin O-Latex-Reagenz und Verfahren zu seiner Herstellung.**

(30) Priorität: **19.03.82 DE 3210080**

(43) Veröffentlichungstag der Anmeldung: **28.09.83 Patentblatt 83/39**

(45) Bekanntmachung des Hinweises auf die Patenterteilung: **08.01.86 Patentblatt 86/2**

(84) Benannte Vertragsstaaten: **AT BE CH DE FR GB IT LI LU NL SE**

(56) Entgegenhaltungen:
**EP-A-0 026 007**
**EP-A-0 043 608**
**DE-A-1 914 081**
**FR-A-2 435 020**
**GB-A-1 531 530**

(73) Patentinhaber: **BEHRINGWERKE Aktiengesellschaft, Postfach 1140, D-3550 Marburg 1 (DE)**

(72) Erfinder: **Toth, Tibor, Dr., Gründeberg 1, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

**EP 0 089 598 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Latex-Reagenz zur Bestimmung von Antikörpern gegen Streptolysin O sowie ein Verfahren zur Herstellung eines solchen Reagenzes.

Streptolysin O ist ein extrazelluläres Stoffwechselprodukt von β-hämolysierenden Streptokokken der Lancefield-Gruppen A, C humanus und G. Es bewirkt im Menschen die Bildung spezifischer Antikörper. Der Nachweis und die Kenntnis der Konzentration dieser Antikörper im menschlichen Blut ist von diagnostischer Bedeutung, da eine erhöhte Konzentration auf eine bestehende oder vorausgegangene Streptokokkeninfektion hindeutet und ein diagnostischer Hinweis auf eine durch Streptokokken verursachte Spondylitis ankylosans (Bechterewsche Krankheit), Glomerulonephritis, Angina, Scharlach, Erysipel, Tonsillitis, Pneumonie oder Sepsis sein kann. Extrem niedrige Anti-Streptolysin O-Titer wurden bei Nephrose und Hypogammaglobulinämie gefunden.

Zur Bestimmung von Anti-Streptolysin O sind bereits Latex-Agglutinationsteste bekannt.

Die bekannten Latex-Reagenzien sind jedoch unter Praxisbedingungen nicht stabil genug und können schnell an Empfindlichkeit verlieren oder zunehmen.

Es wurde nun überraschend ein Verfahren gefunden, das die Herstellung eines stabilen Latex-Reagenzes zur Bestimmung von Anti-Streptolysin O ermöglicht.

Es besteht darin, dass man Streptolysin O mit einer bifunktionellen niedermolekularen Verbindung und gegebenenfalls einem Gammaglobulin oder einem Fab-Fragment eines Gammaglobulins reagieren lässt und das Produkt nach einem bekannten Verfahrn an ein Latex adsorbiert.

Gegenstand der Erfindung ist demnach ein Verfahren zur Herstellung eines Latexreagenzes zur Bestimmung von Antikörpern, die gegen Streptolysin O gerichtet sind, dadurch gekennzeichnet, dass man Streptolysin O mit einer bifunktionellen niedermolekularen Verbindung und gegebenenfalls einem Gammaglobulin oder einem Fab-Fragment eines Gammaglobulins reagieren lässt und das Produkt nach einem bekannten Verfahren an einen Träger, bevorzugt ein Latex, adsorbiert.

Gegenstant der Erfindung ist weiterhin ein Mittel, das ein solches Reagenz enthält.

Die Bindung von Streptolysin O an ein Gammaglobulin oder den Fab-Teil eines solchen ändert die Bindungseigenschaften für den entsprechenden Antikörper nicht. Die Produkte eignen sich für die Herstellung eines stabilen Latex-Reagenzes. Als Gammaglobuline eignen sich im Sinne der Erfindung tierische Gammaglobuline wie beispielsweise Rinder-Gammaglobulin, dessen Fab-Fragmente oder Fab-Fragmente von menschlichen Gammaglobulinen.

Als reaktioinsfähige bifunktionelle Verbindungen kommen solche in Frage, die mit funktionellen Gruppen in Proteinen Bindungen eingehen. Bevorzugt werden Dialdehyde, Bis-oxirane, Diazoniumsalze von Vinylsulfonen, wie beispielsweise aus 1-Aminobenzol-4-β-oxethylsulfonschwefelsäureester erhältlich, Bis-Diazoniumsalze oder wasserlösliche Carbodiimide der allgemeinen Formeln

$OHC-(CH_2)_n-CHO$ $n = 2-4$

Anion $^{-+}N_2-C_6H_4-NH-C_6H_4-N_2^+$ Anion$^-$

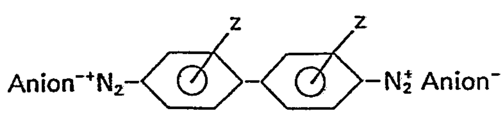

$z = -OCH_3, -COOH$

Anion$^{-+}N_2(CH_2)_6N_2^+$ Anion$^-$

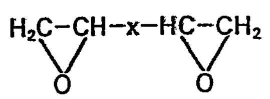

$x = -CH_2, -OCH_2-CH_2-CH_2O-$

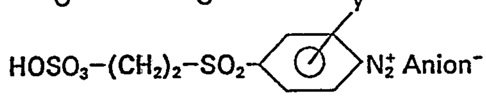

$y = -OCH_3, -CH_3$

$R^1-N=C=N-R^2$

$R^1 =$ 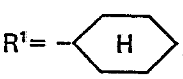 $, -CH_3,$

$-CH_2-CH_2-C_6H_5$

$R^2 = -(CH_2)_3-N(CH_3)_2.HCl,$

$-CH_2CH_2$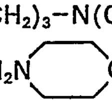$.H_3C\ C_6H_4SO_3H$

Besonders bevorzugt wird jedoch der Glutardialdehyd. Mit der Verbindung, die der sechsten Formel oben entspricht, wobei y = H ist, wurde in der deutschen Offenlegungsschrift Nr. 1914081 Streptolysin O an ein Polysaccharid gekuppelt, und dieser Komplex an Polystyrollatex adsorbiert.

Vorteilhafte Verfahren sind:

Zur Herstellung eines Produktes aus Streptolysin, Gammaglobulin oder seinem Fab-Fragment und bifunktionellem Reagenz wird das Gammaglobulin oder Fab-Fragment mit 1-10 Gewichtsteilen bifunktionellem Reagenz versetzt und die Mischung 2-5 Stunden bei pH 7-10 und 0-20° C gerührt. Das überschüssige Reagenz wird abgetrennt, Glutardialdehyd vorzugsweise mittels einer Sephadex®G 25 oder Sephadex®G 50 Säule, und das Produkt mit 1-5 Gewichtsteilen Streptolysin O bei pH 7-10 und 0-30° C umgesetzt.

Besonders vorteilhaft ist die Umsetzung in einer «Einschritt-Reaktion», indem man die Komponenten Gammaglobulin oder Fab-Fragment mit Streptolysin O in einem Verhältnis 1:0,1 bis 10 mit einer bifunktionellen Verbindung mischt und bei 0-20° C 1-10 Stunden rührt. Im Falle eines Aldehyds als bifunktioneller Verbindung können die nicht umgesetzten Aldehyd-Gruppen in bekannter Weise mit einer Aminosäure, wie beispielsweise Glycin, abgesättigt werden.

Das Reaktionsprodukt wird mit einer Latex-Suspension vermischt, um das erfindungsgemässe Reagenz zu erhalten.

Als Latices sind die an sich bekannten Polymerlatices geeignet, besonders Polystyrollatex.

Das erhaltene Reagenz ist am beständigsten, wenn man Streptolysin O mit einem bifunktionellen Reagenz und einem Gammaglobulin oder einem Fab-Fragment eines solchen reagieren lässt. Doch wird die Stabilität auch erhöht, wenn die Gammaglobulin- oder Fab-Komponente in der Reaktion fehlt.

Das nach dem erfindungsgemässen Verfahren hergestellte Reagenz ist nicht nur stabiler bei Raumtemperatur, sondern auch unter Temperaturbelastung.

*Beispiel 1*

10 g Streptolysin O und 2 g Fab-Fragment eines Gammaglobulins werden mit 0,5 l destilliertem Wasser vermischt und gerührt. Nach etwa 30 Minuten bei 4° C war das Streptolysin O gelöst. Es wurde bei 10000 U/min zentrifugiert und das Sediment verworfen.

Zu der Lösung wurden 4 ml einer 25%igen Glutardialdehydlösung (w:v), die in 900 ml PBS (phosphatgepufferte Kochsalzlösung) gelöst worden war, gegeben und 5 Stunden bei 4° C gerührt. Das Konjugat wurde mit 1 g Glycin versetzt und weitere 12-16 Stunden bei 4° C gerührt.

Die Herstellung des Latex-Reagenzes erfolgte nach bekannten Verfahren. Dazu wurde das Konjugat mit Rinder- oder Human-Albumin vermischt und Polystyrol-Latex zugegeben, bis die gewünschte Empfindlichkeit erreicht war, die mit Hilfe von Verdünnungen eines Standards eingestellt wurde. Mit dem auf diese Weise hergestellten Latex-Reagenz wurde Anti-Streptolysin O im Blut nachgewiesen. Es wurde auch in einer halbquantitativen Bestimmung von Antikörpern gegen Streptolysin O im Röhrchen nach bekannten Verfahren verwendet.

*Beispiel 2*

10 g Streptolysin O und 4 g Fab-Fragment eines Gammaglobulins wurden mit 0,5 l destilliertem Wasser vermischt und gerührt. Nach etwa 30 min bei 4° C war das Streptolysin O gelöst. Es wurde mit 10000 U/min zentrifugiert und das Sediment verworfen. Zu der Lösung wurden 5 ml einer 25%igen Glutardialdehydlösung, die in 800 ml PBS gelöst worden war, gegeben und 5 Stunden bei 4° C gerührt. Der überschüssige Glutardialdehyd wurde über eine Sephadex ®G 50 Säule abgetrennt und das Konjugat nach Konzentrieren für die Herstellung von Latex-ASL-Reagenz eingesetzt.

*Beispiel 3*

5 g Streptolysin O wurden mit 250 ml destilliertem Wasser vermischt und gerührt.

Nach 30 min bei 4° C war das Streptolysin O gelöst. Es wurde bei 10000 U/min zentrifugiert und das Sediment verworfen. Zu der Lösung wurden 69 ml einer 25%igen Glutardialdehydlösung (w:v), die in 300 ml PBS gelöst worden war, gegeben und 2 Stunden bei 4° C gerührt. Der überschüssige Glutardialdehyd wurde über eine Sephadex ®G 50 Säule abgetrennt.

Das Eluat wurde auf etwa 600 ml konzentriert. Zu der Lösung wurden 2 g Fab-Fragment, die in 100 ml PBS gelöst worden waren, gegeben und 12-16 Stunden bei 4° C gerührt. Es wurde 1 g Glycin zu der Lösung gegeben und weitere 16 Stunden gerührt. Das Konjugat wurde zur Herstellung eines Latex-Reagenzes eingesetzt.

*Beispiel 4*

2 g Fab-Fragment eines Gammaglobulins wurden bei +4° C in 80 ml destilliertem Wasser gelöst. Zu der Lösung wurde eine Mischung aus 4 ml einer Lösung von 25 g Glutardialdehyd in 100 ml Wasser und 1500 ml PBS (phosphatgepufferte Kochsalzlösung) gegeben und 5 Stunden bei +4° C gerührt. Der überschüssige Glutardialdehyd wurde über eine Sephadex®G 50 Säule abgetrennt und das Umsetzungsprodukt aus Fab-Fragment und Glutardialdehyd mittels Ultrafiltration konzentriert. Zu der Lösung wurden 10 g Streptolysin O in 500 ml PBS gegeben und 16 Stunden bei +4° C gerührt. Das Konjugat wurde für die Herstellung von Latex-ASL eingesetzt.

*Beispiel 5*

2 g Fab-Fragment eines Gammaglobulins wurden bei +4° C in 150 ml destilliertem Wasser gelöst. Zu der Lösung wurde eine Mischung aus 2 ml einer Lösung von 25 g Glutardialdehyd in 100 ml Wasser und 1500 ml PBS gegeben und die Lösung 5 Stunden bei +4° C gerührt. Es wurden 6 g Streptolysin O, die in 600 ml destilliertem Wasser, das 3,6 g $MgSO_4$ 7 $H_2O$ enthielt, gelöst waren, hinzugefügt und die Mischung 16 Stunden bei +4° C gerührt. Nach Konzentrieren wurde das Konjugat für die Herstellung von Latex-ASL-Reagenz eingesetzt.

Wurden in den voranstehenden Beispielen der Dialdehyd durch 0,1 bis 10 Gewichtsteile 1-Aminobenzol-4-β-oxethyl-sulfonschwefelsäureester(Parabaseester) oder 1-Cyclohexyl-3(2-morpholinoethyl)-carbodiimid p-Toluolsulfonsäure ersetzt, wurden Reagenzien mit vergleichbaren Eigenschaften erhalten. Die Reaktionsbedingungen (pH, Temperatur) wurden dem jeweils verwendeten Reagenz angepasst.

## Patentansprüche

1. Verfahren zur Herstellung eines Antistreptolysin O-Latex-Reagenzes, dadurch gekennzeichnet, dass man das Streptolysin O mit einer bifunktionellen niedermolekularen Verbindung und gegebenenfalls einem Gammaglobulin oder einem Fab-Fragment eines Gammaglobulins reagieren lässt und das Produkt an ein Latex adsorbiert.

2. Verfahren nach Anspruch 1, dadurch gekennzeichnet, dass man ein Fab-Fragment eines Gammaglobulins mit einer bifunktionellen niedermolekularen Verbindung reagieren lässt, die bifunktionelle niedermolekulare Verbindung abtrennt, Streptolysin O zugibt und das Produkt an ein Latex adsorbiert.

3. Verfahren nach Anspruch 1 oder 2, dadurch gekennzeichnet, dass das Latex ein Polystyrol-Homo- oder Polystyrol-Copolymer ist.

4. Verfahren nach einem der Ansprüche 1 bis 3, dadurch gekennzeichnet, dass das Gammaglobulin Human-Gammaglobulin ist.

5. Verfahren nach einem der Ansprüche 1 bis 3, dadurch gekennzeichnet, dass das Fab-Fragment das Fragment von Human-Gammaglobulin ist.

6. Verfahren nach einem der Ansprüche 1 bis 3, dadurch gekennzeichnet, dass das Gammaglobulin Rinder-Gammaglobulin ist.

7. Verfahren nach einem der Ansprüche 1 bis 6, dadurch gekennzeichnet, dass die bifunktionelle Verbindung eine Verbindung nach einer der allgemeinen Formeln

$OHC-(CH_2)_n-CHO$    $n = 2-4$

$Anion^{-+}N_2-C_6H_4-NH-C_6H_4-N_2^+ Anion^-$

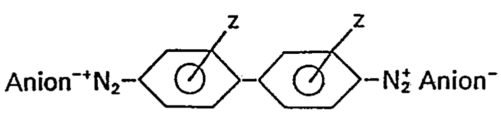
$z = -OCH_3, -COOH$

$Anion^{-+}N_2(CH_2)_6N_2^+ Anion^-$

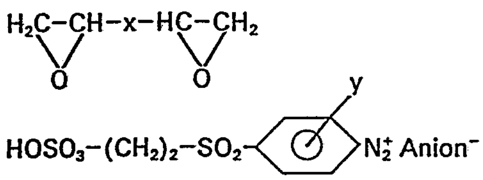
$x = -CH_2, -OCH_2-CH_2-CH_2O-$

$y = -OCH_3, -CH_3$

$R^1-N=C=N-R^2$

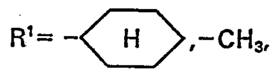

$-CH_2-CH_2-C_6H_5$

$R^2 = -(CH_2)_3-N(CH_3)_2.HCl,$

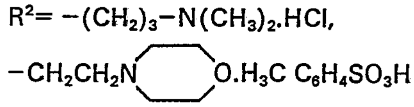

ist.

8. Verfahren nach einem der Ansprüche 1 bis 6, dadurch gekennzeichnet, dass die bifunktionelle Verbindung Glutardialdehyd ist.

9. Mittel, dadurch gekennzeichnet, dass es ein Produkt nach einem der Ansprüche 1 bis 8 enthält.

## Claims

1. A process for the preparation of an antistreptolysin O latex reagent, wherein streptolysin O is reacted with a bifunctional low molecular weight compound and optionally a gamma globulin or a Fab fragment thereof, and the product is adsorbed to a latex.

2. The process as claimed in Claim 1, which comprises reacting a Fab fragment of a gamma globulin with a bifunctional low molecular weight compound, separating the bifunctional low molecular weight compound, adding streptolysin O and adsorbing the product to a latex.

3. The process as claimed in anyone of Claims 1 to 2, wherein the latex is a poystyrene homopolymer or polystyrene copolymer.

4. The process as claimed in anyone of Claims 1 to 3, wherein the gamma globulin is human gamma globulin.

5. The process as claimed in anyone of Claims 1 to 3, wherein the Fab fragment ist the Fab fragment of human gamma globulin.

6. The process as claimed in anyone of Claims 1 to 3, wherein the gamma globulin is bovine gamma globulin.

7. The process as claimed in anyone of Claims 1 to 6, wherein the bifunctional compound is a compound according to one of the formulae

$OHC-(CH_2)_n-CHO$ $n = 2-4$

Anion $^{-+}N_2-C_6H_4-NH-C_6H_4-N_2^+$ Anion$^-$

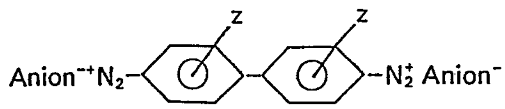

$z = -OCH_3, -COOH$

Anion$^{-+}N_2(CH_2)_6N_2^+$ Anion$^-$

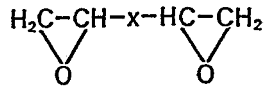

$x = -CH_2, -OCH_2-CH_2-CH_2O-$

$HOSO_3-(CH_2)_2-SO_2-$ 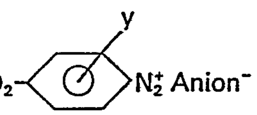

$y = -OCH_3, -CH_3$

$R^1-N=C=N-R^2$

$R^1 =$ 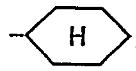 $, -CH_3,$

$-CH_2-CH_2-C_6H_5$

$R^2 = -(CH_2)_3-N(CH_3)_2.HCl,$

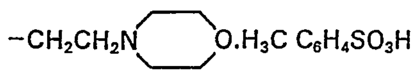

8. The process as claimed in anyone of Claims 1 to 6, wherein the bifunctional compound is glutaric dialdehyde.

9. Agent which comprises a product according to anyone of Claims 1 to 8.

## Revendications

1. Procédé de préparation d'un réactif au latex, antistreptolysine O, caractérisé en ce qu'on fait réagir la streptolysine O avec un composé bifonctionnel de faible masse moléculaire et éventuellement une gamma-globuline ou un fragment Fab d'une gamma-globuline et on adsorbe le produit sur un latex.

2. Procédé selon la revendication 1, caractérisé en ce qu'on fait réagir un fragment Fab d'une gamma-globuline avec un composé bifonctionnel de faible masse moléculaire, on sépare le composé bifonctionnel de faible masse moléculaire, on ajoute la streptolysine O et on adsorbe le produit sur un latex.

3. Procédé selon la revendication 1 ou 2, caractérisé en ce que le latex est un homopolymère ou un copolymère de polystyrène.

4. Procédé selon l'une des revendications 1 à 3, caractérisé en ce que la gamma-globuline est une gamma-globuline humaine.

5. Procédé selon l'une des revendications 1 à 3, caractérisé en ce que le fragment Fab est le fragment d'une gamma-globuline humaine.

6. Procédé selon l'une des revendications 1 à 3, caractérisé en ce que la gamma-globuline est une gamma-globuline de bœuf.

7. Procédé selon l'une quelconque des revendications 1 à 6, caractérisé en ce que le composé bifonctionnel est un composé selon l'une des formules générales:

$OHC-(CH_2)_n-CHO$ $n = 2-4$

Anion $^{-+}N_2-C_6H_4-NH-C_6H_4-N_2^+$ Anion$^-$

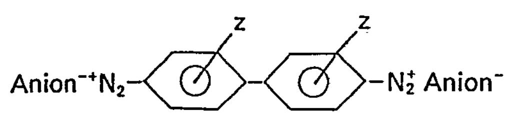

$z = -OCH_3, -COOH$

Anion$^{-+}N_2(CH_2)_6N_2^+$ Anion$^-$

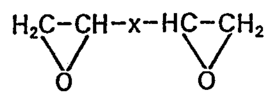

$x = -CH_2, -OCH_2-CH_2-CH_2O-$

$HOSO_3-(CH_2)_2-SO_2-$ 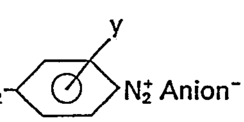

$y = -OCH_3, -CH_3$

$R^1-N=C=N-R^2$

$R^1 =$ 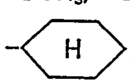 $, -CH_3,$

$-CH_2-CH_2-C_6H_5$

$R^2 = -(CH_2)_3-N(CH_3)_2.HCl,$

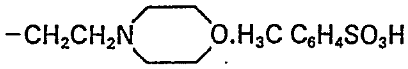

8. Procédé selon l'une des revendications 1 à 6, caractérisé en ce que le composé bifonctionnel est le dialdéhyde glutarique.

9. Agent caractérisé en ce qu'il contient un produit selon l'une des revendications 1 à 8.